Europäisches Patentamt

European Patent Office

Office européen des brevets

(19)

(11) Veröffentlichungsnummer : **0 424 736 B1**

# (12) EUROPÄISCHE PATENTSCHRIFT

(45) Veröffentlichungstag der Patentschrift :
**15.12.93 Patentblatt 93/50**

(21) Anmeldenummer : **90119472.0**

(22) Anmeldetag : **11.10.90**

(51) Int. Cl.$^5$ : **C01G 55/00,** B01J 31/40,
C22B 3/00

(30) Priorität : **19.10.89 DE 3934824**

(43) Veröffentlichungstag der Anmeldung :
**02.05.91 Patentblatt 91/18**

(45) Bekanntmachung des Hinweises auf die
Patenterteilung :
**15.12.93 Patentblatt 93/50**

(84) Benannte Vertragsstaaten :
**AT BE CH DE ES FR GB IT LI NL SE**

(56) Entgegenhaltungen :
**EP-A- 0 210 017**

(56) Entgegenhaltungen :
**EP-A- 0 255 673**
**EP-A- 0 322 661**
**EP-A- 0 348 833**
**US-A- 4 390 473**

(73) Patentinhaber : **HOECHST**
**AKTIENGESELLSCHAFT**
**D-65926 Frankfurt (DE)**

(72) Erfinder : **Lappe, Peter, Dr. Dipl.-Chem.**
**Eickenhof 34**
**W-4220 Dinslaken (DE)**
Erfinder : **Springer, Helmut, Dipl.-Ing.**
**Borbecker Strasse 19**
**W-4200 Oberhausen 11 (DE)**

(54) **Verfahren zur Wiedergewinnung von Rhodium aus den Rückständen der Destillation von Produkten der Oxosynthese.**

## Beschreibung

Die vorliegende Erfindung betrifft ein verbessertes Verfahren zur Wiedergewinnung von Rhodium aus den Rückständen, die bei der Destillation von Produkten der Oxosynthese erhalten werden.

Die Herstellung von Aldehyden und Alkoholen durch Anlagerung von Kohlenmonoxid und Wasserstoff an olefinische Doppelbindungen (Hydroformylierung) ist bekannt. Die Reaktion wird durch Metalle der 8. Nebengruppe des Periodensystems oder deren Verbindungen katalysiert, die unter den Reaktionsbedingungen Carbonyle oder Hydridocarbonyle bilden. Während früher fast ausschließlich Kobalt und Kobaltverbindungen als Katalysatoren eingesetzt wurden, finden heute in zunehmendem Maße Rhodiumkatalysatoren Anwendung, obgleich Rhodium um ein vielfaches teurer als Kobalt ist. Rhodium gelangt dabei allein oder in Kombination mit Komplexbildnern, z.B. organischen Phosphinen, zum Einsatz. Während die Oxosynthese mit Rhodium als Katalysator Reaktionsdrücke von 25 bis 30 MPa erfordert, genügen bei Einsatz von Rhodium-Komplexverbindungen Drücke von 1 bis 5 MPa.

Für Rhodium-Katalysatoren ergeben sich in vielen Fällen deutliche Vorteile. Sie besitzen höhere Aktivität und Selektivität und ermöglichen überdies einen in vieler Hinsicht problemlosen Betrieb der Produktionsanlage, insbesondere was die Durchführung der Synthese und die Ausbringung der Produkte aus dem Reaktor anbetrifft. Schließlich kann das klassische Oxoverfahren auf Basis von Kobaltkatalysatoren unter Verwendung der vorhandenen Apparateteile in vielen Fällen mit nur geringen Investitionen auf Rhodiumkatalysatoren umgestellt werden.

Erhebliche Schwierigkeiten bereitet jedoch die verlustfreie oder zumindest annähernd verlustfreie Abtrennung und Wiedergewinnung des Rhodiums, unabhängig davon, ob es mit oder ohne zusätzlichem Komplexbildner als Katalysator eingesetzt wird. Nach Beendigung der Umsetzung findet sich das Rhodium als Carbonylverbindung, die gegebenenfalls noch weitere Liganden enthalten kann, gelöst im Hydroformylierungsprodukt.

Zur Aufarbeitung wird das Oxorohprodukt üblicherweise mehrstufig entspannt, indem man den Synthesedruck, der je nach Art des eingesetzten Rhodiumkatalysators etwa 1 bis 30 MPa beträgt, zunächst auf etwa 0,5 bis 2,5 MPa reduziert. Hierbei wird im Rohprodukt gelöstes Synthesegas frei. Anschließend kann man auf Normaldruck entspannen. Die Abtrennung des Rhodiums erfolgt entweder unmittelbar aus dem Rohprodukt oder aus dem Rückstand der Rohproduktdestillation. Der erste Weg wird dann beschritten, wenn in der vorausgegangenen Hydroformylierungsstufe Rhodium ohne zusätzlichen Komplexbildner als Katalyator eingesetzt worden war. Die zweite Variante wird angewandt, wenn der Rhodiumkatalysator außer Kohlenmonoxid noch weitere Liganden, z.B. Phosphine oder Phosphite in komplexer Bindung enthält. Sie kann auch dann genutzt werden, wenn die Hydroformylierung zwar mit Rhodium allein durchgeführt, dem Rohprodukt nach Entspannung aber ein Komplexbildner zur Stabilisierung des Rhodiums zugesetzt worden war. Grundsätzlich ist zu berücksichtigen, daß das Edelmetall im Rohprodukt in einer Konzentration von nur wenigen ppm vorliegt, seine Abtrennung daher sehr sorgfältiges Arbeiten erfordert. Zusätzliche Schwierigkeiten können dadurch entstehen, daß das Rhodium, insbesondere wenn es ohne Ligand eingesetzt wurde, bei der Entspannung teilweise in metallische Form übergeht oder mehrkernige Carbonyle bildet. Es kommt dann zur Ausbildung eines heterogenen Systems, das aus der flüssigen organischen und der festen, Rhodium oder Rhodiumverbindungen enthaltenden Phase besteht.

Die Rückgewinnung von Rhodium aus den Produkten der Oxosynthese, darunter auch den Rückständen von Oxorohprodukten, ist vielfach untersucht worden. Die Arbeiten führten zur Entwicklung zahlreicher Verfahren, von denen einige auch Anwendung im technischen Maßstab gefunden haben.

Gegenstand der US-A-4 400 547 ist die Hydroformylierung von Olefinen mit 2 bis 20 Kohlenstoffatomen in Gegenwart von unmodifiziertem Rhodium als Katalysator. Nach Beendigung der Reaktion setzt man dem Oxorohprodukt eine komplexbildende Verbindung wie Triphenylphosphin zu und destilliert den Aldehyd ab. Anschließend wird der Destillationsrückstand mit Sauerstoff behandelt, um den Liganden aus der Komplexverbindung wieder abzuspalten und das Rhodium in aktiver Form zurückzugewinnen. Eine Trennung von Rhodium und Destillationsrückstand ist nach dieser Arbeitsweise nicht möglich.

Die Abtrennung von Edelmetallen wie Rhodium aus hochsiedenden Hydroformylierungsrückständen wird auch in der US-A-3 547 964 beschrieben. Hierzu behandelt man die Rückstände in Gegenwart von Säuren wie Ameisensäure, Salpetersäure oder Schwefelsäure mit Wasserstoffperoxid. Wegen des hohen Preises von Wasserstoffperoxid und seiner problematischen Handhabung sind der technischen Anwendung des Verfahrens jedoch Grenzen gesetzt.

Nach der DE 24 48 005 C2 wird ein Rhodium enthaltender Destillationsrückstand zunächst mit Säuren und Peroxiden behandelt. Darauf zerstört man überschüssige Peroxide durch Erhitzen und setzt die das Katalysatormetall enthaltende wäßrige Lösung in Gegenwart eines wasserlöslichen organischen Lösungsmittels mit Halogenwasserstoffsäure oder Alkalihalogeniden sowie mit tertiären Phosphinen und Kohlenmonoxid oder Kohlenmonoxid abspaltenden Verbindungen um. Diese Arbeitsweise erfordert wiederum die Verwendung von Peroxiden mit den oben geschilderten Nachteilen und den Einsatz halogenbeständiger Werkstoffe.

In der US-A-4 390 473 schließlich ist ein Verfahren zur Wiedergewinnung von Rhodium und Kobalt aus einer Lösung beschrieben, die als Katalysator in einem Niederdruck-Oxoverfahren eingesetzt worden war. Zur Abtrennung der komplex gebundenen Metalle gibt man zu der Lösung wäßrige Ameisensäure und leitet ein Sauerstoff enthaltendes Gas durch. Es entstehen zwei Phasen, eine organische und eine wäßrige, die die Metalle als Formiate gelöst enthält. Nach Trennung der Phasen können Kobalt und Rhodium aus der wäßrigen Lösung gewonnen werden. In der Praxis hat es sich jedoch als sehr störend erwiesen, daß die Ameisensäure reduzierend wirkt. Diese Eigenschaft führte dazu, daß Rhodium im Laufe des Verfahrens teilweise in metallischer Form abgeschieden wurde und nicht mehr wiedergewonnen werden konnte.

Es bestand daher die Aufgabe ein Verfahren zu entwickeln, das die geschilderten Nachteile vermeidet und auf möglichst einfachem Wege eine verlustfreie oder doch annähernd verlustfreie Wiedergewinnung des Edelmetalls sicherstellt.

Erfindungsgemäß wird diese Aufgabe gelöst durch ein Verfahren zur Wiedergewinnung von Rhodium, das in komplexer Bindung mit einer organischen Phosphor(III)-verbindung in den Rückständen der Destillation von Produkten der Oxosynthese enthalten ist, durch Behandlung dieser Rückstände mit Sauerstoff oder einem Sauerstoff enthaltenden Gas. Es ist dadurch gekennzeichnet, daß die Rückstände mit Sauerstoff oder einem Sauerstoff enthaltenden Gas bei 60 bis 120°C drucklos oder unter Druck in Gegenwart einer $C_2$- bis $C_5$-Monocarbonsäure und des Alkalisalzes einer $C_2$-bis $C_5$-Monocarbonsäure behandelt und anschließend zur Abtrennung des Rhodiums als wasserlösliche Verbindung mit Wasser extrahiert und darauf wäßrige und organische Phase voneinander getrennt werden.

Die erfindungsgemäße Arbeitsweise erfordert keinen großen apparativen Aufwand oder den Einsatz wertvoller Chemikalien. Dennoch führt sie überraschend zur Wiedergewinnung von weit mehr als 90% des eingesetzten Rhodiums. Dabei fällt das Metall in einer Form an, das seinen erneuten Einsatz als Katalysator ohne besondere zusätzliche Maßnahmen ermöglicht.

Das neue Verfahren geht von den Rückständen der Hydroformylierung olefinisch ungesättigter Verbindungen aus, die nach dem Abdestillieren der Aldehyde und als Nebenprodukte gebildeter Alkohole im Destillationssumpf zurückbleiben. Sie bestehen im wesentlichen aus höhermolekularen Verbindungen, die aus den Aldehyden durch Aldolkondensation entstanden sind und in einer Folgereaktion unter Bildung ungesättigter Verbindungen auch Wasser abspalten können. Die Art der Verbindungen, die hydroformyliert wurden, ist für die beanspruchte Arbeitsweise ohne Bedeutung. Dementsprechend können sowohl Rückstände eingesetzt werden, die aus der Reaktion von Olefinen mit Kohlenmonoxid und Wasserstoff resultieren, als auch höhermolekulare Produkte, die bei der Umsetzung olefinisch ungesättigter Verbindungen entstehen, die außer der Doppelbindung noch funktionelle Gruppen im Molekül enthalten. Im Vordergrund des neuen Verfahrens steht aber die Wiedergewinnung von Rhodium aus den Rückständen der Hydroformylierung von Olefinen mit 2 bis 12 Kohlenstoffatomen, entsprechend der wirtschaftlichen Bedeutung der aus ihnen hergestellten Aldehyde. Neben den gesättigten und ungesättigten Kondensationsprodukten können die Rückstände auch noch Verbindungen enthalten, die mit den Rhodiumionen unter Komplexbildung reagieren und gegenüber dem Rhodium zumeist im Überschuß vorhanden sind. Zu diesen Verbindungen gehören organische Phosphor(III)-verbindungen insbesondere Phosphine und Phosphite vorzugsweise die Arylverbindungen wie Triphenylphosphin und Triphenylphosphit. Ihre Aufgabe ist es durch Bildung stabiler Komplexverbindungen während der Umsetzung die Selektivität der Reaktion zu verbessern und nach der Umsetzung die Abscheidung metallischen Rhodiums zu verhindern. Im Reaktionsgemisch betragt das Verhältnis von Ligand und Rhodium bevorzugt 2 bis 150 und insbesondere 5 bis 50 Mol/g-At. Wegen ihrer geringen Flüchtigkeit liegen beide Komponenten auch im Destillationsrückstand etwa im selben Verhältnis vor, wobei die Rhodiumkonzentration zwischen 30 und 1000 ppm, vorzugsweise 100 bis 500 ppm beträgt.

Erfindungsgemäß wird der Destillationsrückstand mit Sauerstoff behandelt. Das Oxidationsmittel wird in reiner Form eingesetzt oder als Sauerstoff enthaltendes Gasgemisch, insbesondere Luft. Die Sauerstoffmenge kann in weiten Grenzen variiert werden. Sie richtet sich vorzugsweise nach der Konzentration der Phosphor-(III)-verbindungen im Rückstand. Es empfiehlt sich je mol Phosphor-(III)-verbindung 100 bis 2000, insbesondere 300 bis 1200 mol Sauerstoff anzuwenden.

Entsprechend der Erfindung erfolgt die Behandlung des Destillationsrückstandes mit Sauerstoff in Gegenwart einer gesättigten, geradkettigen oder verzweigten Monocarbonsäure mit 2 bis 5 Kohlenstoffatomen, Beispiele für geeignete Säuren sind Essigsäure, Propionsäure, n-Buttersäure, i-Buttersäure und n-Valeriansäure. Besonders bewährt haben sich Essigsäure und Propionsäure. Sie werden in der handelsüblichen Form und in einer solchen Menge eingesetzt, daß je g-At Rhodium etwa 10 bis 150, vorzugsweise 10 bis 50 mol vorhanden sind. Die Säure wird dem Rückstand vor der Umsetzung mit Sauerstoff zugesetzt unabhängig davon, daß sich aufgrund von geringen Mengen Aldehyd, die im Rückstand vorhanden sind, im Laufe der Reaktion aus dem Rückstand selbst Säure bilden kann. Die genaue Wirkungsweise der Säure ist nicht bekannt. Verschiedene Beobachtungen sprechen dafür, daß sie eine Startfunktion ausübt, d.h. das Einsetzen der Reaktion maßgebend beeinflußt.

Ein weiteres, ebenfalls sehr wichtiges Merkmal des erfindungsgemäßen Verfahrens ist die Anwesenheit eines Alkalicarboxylats im Rückstand während Sauerstoff in das Gemisch der hochsiedenden Verbindungen eingeleitet wird. Auch die Art seines Eingreifens in das Reaktionsgeschehen ist nicht eindeutig zu erklären. Es hat sich jedoch gezeigt, daß der Carboxylatzusatz eine deutliche Erhöhung der zurückgewonnenen Rhodiummenge d.h. eine weitere Verminderung des in der organischen Phase gelöst bleibenden Rhodiums zur Folge hat. Als Alkalicarboxylate werden im Rahmen des neuen Verfahrens Salze gesättigter, geradkettiger oder verzweigter Monocarbonsäuren mit 2 bis 5 Kohlenstoffatomen eingesetzt. Besonders bewährt haben sich die Natrium- und Kaliumsalze der Essigsäure, der Propionsäure, der n- und iso-Buttersäure und der n-Valeriansäure. Sie werden in einer Menge von 50 bis 250, vorzugsweise 100 bis 180 mol je g-At Rhodium angewandt. Geeignet sind die handelsüblichen Salze, die jedoch erst im Verlauf der Oxidation allmählich in Lösung gehen. Vorteilhafter ist es daher, zum Rückstand freie Säure und die äquivalente Menge Alkalihydroxid zu geben, die sofort homogen gelöst und damit voll wirksam werden.

Die Umsetzung des Rückstandes mit Sauerstoff erfolgt bei 60 bis 120, vorzugsweise 80 bis 100°C. Sie kann drucklos oder unter Druck vorgenommen werden, Drücke zwischen 0,2 und 1,0 MPa haben sich besonders bewährt.

Nach einer bevorzugten Ausführungsform des erfindungsgemäßen Verfahrens enthalten die mit Sauerstoff zu behandelnden Rückstände Rhodium in einer Konzentration von etwa 100 ppm und weniger, vorzugsweise 30 bis 90 ppm. Es wurde nämlich gefunden, daß die Rhodiumrestmengen im erfindungsgemäßen behandelten Rückstand dann besonders niedrig sind, wenn die Metallkonzentration in der Ursprungslösung innerhalb der genannten Bereiche liegt. Es empfiehlt sich daher Lösungen, in denen die Rhodiumkonzentration mehr als etwa 100 ppm beträgt entsprechend zu verdünnen. Als Verdünnungsmittel geeignet sind insbesondere höher siedende aliphatische oder aromatische Kohlenwasserstoffe oder Kohlenwasserstoffgemische wie Toluol und Xylol oder auch vom Rhodium-Katalysator befreite Destillationsrückstände.

Die Reaktionszeit ist abhängig von der Rhodium- und der Ligandenkonzentration im Destillationsrückstand. Sie wird weiterhin durch die eingesetzte Sauerstoffmenge, durch Reaktionstemperatur und -druck bestimmt. Hohe Konzentrationen der gelösten Substanzen erfordern längere Behandlungszeiten als niedrige Konzentrationen. Ein großes Sauerstoffangebot und erhöhter Druck vermindern die Reaktionszeit ebenso wie eine intensive Durchmischung des Rückstandes mit Sauerstoff. Temperaturen im unteren und im oberen Bereich des beanspruchten Intervalls sind etwas weniger effektiv als im mittleren Temperaturbereich.

Die Umsetzung des Destillationsrückstandes kann in konventionellen Apparaturen kontinuierlich oder diskontinuierlich durchgeführt werden. Der Sauerstoff oder das Sauerstoff enthaltende Gas wird über Verteilungsvorrichtungen in den Reaktor geleitet, die gleichmäßige Vermischung von flüssiger und gasförmiger Phase gegebenenfalls durch Rühren unterstützt.

Nach Abschluß der Behandlung mit Sauerstoff wird die organische Phase mit Wasser extrahiert. Man arbeitet bei Raumtemperatur oder wenig erhöhter Temperatur in einer, zweckmäßiger in mehreren Stufen. Die eingesetzte Wassermenge richtet sich nach dem Verteilungsgleichgewicht der zu extrahierenden Substanz zwischen organischer und wäßriger Phase und der angestrebten Rhodiumkonzentration in der wäßrigen Phase. Die wäßrige Lösung der Rhodiumverbindung kann auch durch Kreisführung wiederholt zur Extraktion verwendet werden um dadurch eine Anreicherung des Metalles in der Lösung zu erzielen. Die wäßrige Lösung kann ohne zusätzliche Reinigungsschritte unmittelbar zur Katalysatorherstellung verwendet werden.

Die folgenden Beispiele erläutern die Erfindung. Es ist aber selbstverständlich nicht beabsichtigt, sie auf diese Ausführungsformen zu beschränken.

In Tabelle 1 sind die Einsatzstoffe durch ihre wesentlichen Kennzahlen charakterisiert.

## Tabelle 1

|  | Produkt A | Produkt B | Produkt C | Produkt D |
|---|---|---|---|---|
| Rh-Gehalt (ppm) | 117 | 142 | 224 | 226 |
| P(III) (mmol/kg) | 2,5 | 4,8 | 15,0 | 7,7 |
| P ges. (mmol/kg) | 7,2 | 6,3 | 21,1 | 13,4 |
| CO-Zahl (mg KOH/g) | 247 | 300 | 368 | 351 |
| Dichte 20°C (g/cm$^3$) | 0,952 | 0,961 | 0,949 | 0,942 |
| Wasser (%) | 0,09 | 0,05 | 0,09 | 0,22 |

### Beispiel 1

In einem mit Mantelbeheizung versehenen l 1-Glasautoklaven werden 300,0 g Destillationsrückstand B, 300,0 g Xylol, 8,22 g 30 %ige Natronlauge sowie 5,18 g 99,5 %ige Propionsäure vorgelegt und unter Rühren innerhalb von 15 Minuten auf 78°C erwärmt. Anschließend leitet man durch ein Tauchrohr über einen Zeitraum von 5 h und unter einem Druck von 0,2 MPa je Stunde 20 l Luft ein.

Die Umsetzung erfolgt bei konstantem Innendruck von 0,25 MPa und konstanter Temperatur von 80°C.

Das Abgas wird über ein Nadelventil im Deckel des Autoklaven entspannt und in einen mit Kühler versehenen Kolben geleitet.

Nach Beendigung der Reaktion kühlt man den Autoklaveninhalt in etwa 15 Minuten auf 60°C und stellt die Luftzufuhr ein. Anschließend wird entspannt, das Reaktionsgemisch mit 200,0 g Wasser versetzt und weitere 15 Minuten bei 50 bis 60°C gerührt. Man entnimmt das Produkt dem Reaktor, trennt die Phasen und extrahiert die organische Phase noch zweimal mit je 200,0 g Wasser. Nach der Behandlung enthält die organische Phase noch 1,9 mg Rh entsprechend 4,4 % des im Einsatzstoff enthaltenen Rh.

### Beispiele 2 bis 19

Die Versuchsdurchführung erfolgt analog Beispiel 1. Einsatzstoffe, Reaktionsbedingungen und Ergebnisse sind in Tabelle 2 zusammengestellt.

Tabelle 2:

| Beispiel-Nr. | Einsatzstoff Art | Menge (g) | Xylol (g) | Temp. (°C) | t (h) | p (MPa) | Na-carboxylat Art | Menge (mol/g-At Rh) | Carbonsäure Art | Menge (mol/g-At Rh) | Luftmenge (l/Std. mMol P(III)$^{-1}$) | Rh-Gehalt in der org. Phase (% bez. auf Einsatz) |
|---|---|---|---|---|---|---|---|---|---|---|---|---|
| 2 | B | 400 | 200 | 80 | 5 | 0,25 | $C_3$ | 150 | $C_3$ | 50 | 14 | 5,6 |
| 3 | B | 300 | 300 | 80 | 5 | 0,25 | $C_3$ | 150 | $C_3$ | 50 | 14 | 4,1 |
| 4 | B | 240 | 360 | 80 | 5 | 0,25 | $C_3$ | 150 | $C_3$ | 50 | 14 | 4,1 |
| 5 | A | 360 | 240 | 80 | 5 | 0,25 | $C_2$ | 150 | $C_2$ | 20 | 17 | 4,7 |
| 6 | A | 360 | 240 | 80 | 5 | 0,25 | $C_3$ | 150 | $C_3$ | 20 | 17 | 4,3 |
| 7 | A | 360 | 240 | 80 | 5 | 0,25 | $i\text{-}C_4$ | 150 | $i\text{-}C_4$ | 20 | 17 | 5,2 |
| 8 | D | 175 | 425 | 80 | 5 | 0,25 | $C_3$ | 150 | $C_3$ | 20 | 70 | 8,0 |
| 9 | D | 175 | 425 | 85 | 5 | 0,25 | $C_3$ | 150 | $C_3$ | 20 | 70 | 6,9 |
| 10 | D | 175 | 425 | 90 | 5 | 0,25 | $C_3$ | 150 | $C_3$ | 20 | 70 | 8,2 |
| 11 | C | 188 | 412 | 80 | 5 | 0,25 | $C_3$ | 150 | $C_3$ | 20 | 14 | 4,3 |
| 12 | B | 300 | 300 | 80 | 5 | 0,25 | $C_3$ | 150 | $C_3$ | 10 | 14 | 5,0 |
| 13 | B | 300 | 300 | 80 | 5 | 0,25 | $C_3$ | 150 | $C_3$ | 20 | 14 | 4,7 |
| 14 | B | 300 | 300 | 80 | 5 | 0,25 | $C_3$ | 150 | $C_3$ | 50 | 14 | 4,1 |
| 15 | B | 300 | 300 | 80 | 5 | 0,25 | $C_3$ | 50 | $C_3$ | 20 | 14 | 4,3 |
| 16 | B | 300 | 300 | 80 | 5 | 0,25 | C3 | 100 | $C_3$ | 20 | 14 | 4,0 |
| 17 | B | 300 | 300 | 80 | 5 | 0,25 | $C_3$ | 150 | $C_3$ | 20 | 14 | 3,9 |
| 18 | D | 175 | 425 | 80 | 5 | 0,25 | $C_3$ | 150 | $C_3$ | 20 | 25 | 6,9 |
| 19 | D | 175 | 425 | 80 | 5 | 0,25 | $C_3$ | 150 | $C_3$ | 20 | 15 | 7,2 |

EP 0 424 736 B1

**Patentansprüche**

1. Verfahren zur Wiedergewinnung von Rhodium, das in komplexer Bindung mit einer organischen Phosphor-(III)-verbindung in den Rückständen der Destillation von Produkten der Oxosynthese enthalten ist durch Behandlung dieser Rückstände mit Sauerstoff oder einem Sauerstoff enthaltenden Gas dadurch gekennzeichnet, daß die Rückstände mit Sauerstoff oder einem Sauerstoff enthaltenden Gas bei 60 bis 120°C drucklos oder unter Druck in Gegenwart einer $C_2$- bis $C_5$-Monocarbonsäure und des Alkalisalzes einer $C_2$- bis $C_5$-Monocarbonsäure behandelt und anschließend zur Abtrennung des Rhodiums als wasserlösliche Verbindung mit Wasser extrahiert und darauf wäßrige und organische Phase voneinander getrennt werden.

2. Verfahren nach Anspruch 1 dadurch gekennzeichnet, daß als Sauerstoff enthaltendes Gas Luft verwendet wird.

3. Verfahren nach Anspruch 1 oder 2 dadurch gekennzeichnet, daß je mol Phosphor-(III)-verbindung 100 bis 2000, insbesondere 300 bis 1200 mol Sauerstoff angewendet werden.

4. Verfahren nach einem oder mehreren der Ansprüche 1 bis 3 dadurch gekennzeichnet, daß den Rückständen Essigsäure, Propionsäure, n-Buttersäure, i-Buttersäure oder n-Valeriansäure zugesetzt werden.

5. Verfahren nach einem oder mehreren der Ansprüche 1 bis 4 dadurch gekennzeichnet, daß den Rückständen je g-At Rhodium 10 bis 150, vorzugsweise 10 bis 50 mol Monocarbonsäure zugesetzt werden.

6. Verfahren nach einem oder mehreren der Ansprüche 1 bis 5 dadurch gekennzeichnet, daß den Rückständen ein Natrium- oder Kaliumsalz der Essigsäure, Propionsäure, n-Buttersäure, i-Buttersäure oder n-Valeriansäure zugesetzt wird.

7. Verfahren nach einem oder mehreren der Ansprüche 1 bis 6 dadurch gekennzeichnet, daß den Rückständen je g-At Rhodium 50 bis 250, vorzugsweise 100 bis 180 mol Alkalisalz einer $C_2$- bis $C_5$-Monocarbonsäure zugesetzt wird.

8. Verfahren nach einem oder mehreren der Ansprüche 1 bis 7 dadurch gekennzeichnet, daß die Behandlung der Rückstände mit Sauerstoff oder einem Sauerstoff enthaltenden Gas bei 80 bis 100°C erfolgt.

9. Verfahren nach einem oder mehreren der Ansprüche 1 bis 8 dadurch gekennzeichnet, daß die Behandlung der Rückstände mit Sauerstoff oder einem Sauerstoff enthaltenden Gas bei Drücken von 0,2 bis 1,0 MPa erfolgt.

10. Verfahren nach einem oder mehreren der Ansprüche 1 bis 9 dadurch gekennzeichnet, daß die Rhodiumkonzentration in den Rückständen etwa 100 ppm und weniger, vorzugsweise 30 bis 90 ppm beträgt.

**Claims**

1. A process for the recovery of rhodium which is present in the distillation residues of products of the oxo synthesis bound in the form of a complex to an organic phosphorus(III) compound by treatment of these residues with oxygen or an oxygen-containing gas, comprising treating the residues with oxygen or an oxygen-containing gas at 60 to 120°C at atmospheric or elevated pressure in the presence of a $C_2$- to $C_5$-monocarboxylic acid and the alkali metal salt of a $C_2$- to $C_5$-monocarboxylic acid and then separating the rhodium as a water-soluble compound by extraction with water and then separating the aqueous and organic phase from one another.

2. The process as claimed in claim 1, wherein air is used as the oxygen-containing gas.

3. The process as claimed in claim 1 or 2, wherein 100 to 2000, in particular 300 to 1200, mol of oxygen are used per mole of phosphorus(III) compound.

4. The process as claimed in one or more of claims 1 to 3, wherein acetic acid, propionic acid, n-butyric acid, i-butyric acid or n-valeric acid are added to the residues.

5. The process as claimed in one or more of claims 1 to 4, wherein 10 to 150, preferably 10 to 50, mol of monocarboxylic acid are added to the residues per g-atom of rhodium.

6. The process as claimed in one or more of claims 1 to 5, wherein a sodium or potassium salt of acetic acid, propionic acid, n-butyric acid, i-butyric acid or n-valeric acid is added to the residues.

7. The process as claimed in one or more of claims 1 to 6, wherein 50 to 250, preferably 100 to 180, mol of an alkali metal salt of a $C_2$- to $C_5$-monocarboxylic acid are added to the residues per g-atom of rhodium.

8. The process as claimed in one or more of claims 1 to 7, wherein the treatment of the residues with oxygen or an oxygen-containing gas is carried out at 80 to 100°C.

9. The process as claimed in one or more of claims 1 to 8, wherein the treatment of the residues with oxygen or an oxygen-containing gas is carried out at pressures of 0.2 to 1.0 MPa.

10. The process as claimed in one or more of claims 1 to 9, wherein the rhodium concentration in the residues is about 100 ppm and less, preferably 30 to 90 ppm.


## Revendications

1. Procédé pour récupérer le rhodium contenu à l'état de combinaison complexe avec un composé organique du phosphore-III dans les résidus de la distillation de produits de l'oxosynthèse par traitement de ces résidus par l'oxygène ou un gaz contenant de l'oxygène, caractérisé en ce que l'on traite les résidus par l'oxygène ou un gaz contenant de l'oxygène à des températures de 60 à 120°C à pression normale ou sous pression en présence d'un acide monocarboxylique en $C_2$-$C_5$ et d'un sel alcalin d'un acide monocarboxylique en $C_2$-$C_5$ puis, pour séparer le rhodium à l'état de composé soluble dans l'eau, on extrait par l'eau et on sépare la phase aqueuse et la phase organique.

2. Procédé selon la revendication 1, caractérisé en ce que l'on utilise l'air en tant que gaz contenant de l'oxygène.

3. Procédé selon la revendication 1 ou 2, caractérisé en ce que l'on utilise de 100 à 2 000, plus spécialement de 300 à 1 200 mol d'oxygène par mole de composé du phosphore-III.

4. Procédé selon une ou plusieurs des revendications 1 à 3, caractérisé en ce que l'on ajoute aux résidus de l'acide acétique, de l'acide propionique, de l'acide n-butyrique, de l'acide isobutyrique ou de l'acide n-valérique.

5. Procédé selon une ou plusieurs des revendications 1 à 4, caractérisé en ce que l'on ajoute aux résidus de 10 à 150, de préférence de 10 à 50 mol d'acide monocarboxylique par atome-gramme de rhodium.

6. Procédé selon une ou plusieurs des revendications 1 à 5, caractérisé en ce que l'on ajoute aux résidus un sel de sodium ou de potassium de l'acide acétique, de l'acide propionique, de l'acide n-butyrique, de l'acide isobutyrique ou de l'acide n-valérique.

7. Procédé selon une ou plusieurs des revendications 1 à 6, caractérisé en ce que l'on ajoute aux résidus de 50 à 250, de préférence de 100 à 180 mol de sel alcalin d'un acide monocarboxylique en $C_2$-$C_5$ par atome-gramme de rhodium.

8. Procédé selon une ou plusieurs des revendications 1 à 7, caractérisé en ce que le traitement des résidus par l'oxygène ou un gaz contenant de l'oxygène est réalisé à des températures de 80 à 100°C.

9. Procédé selon une ou plusieurs des revendications 1 à 8, caractérisé en ce que le traitement des résidus par l'oxygène ou un gaz contenant de l'oxygène est réalisé à des pressions de 0,2 à 1,0 MPa.

10. Procédé selon une ou plusieurs des revendications 1 à 9, caractérisé en ce que la concentration en rhodium des résidus est d'environ 100 ppm ou moins, de préférence de 30 à 90 ppm.